# EUROPEAN PATENT APPLICATION

(11) **EP 4 621 036 A1**
(43) Date of publication of application: **24.09.2025**
(21) Application number: 24164044.0
(22) Date of filing: 18.03.2024
(51) Int. Cl.: C11D 3/386, C11D 3/39, C11D 3/16, C11D 3/22, C11D 3/48

(54) **BIODEGADABLE QUADROBIOTECHNOLOGY FOR HYGIENIC CLEANSING OF DISHES AND PREVENTION OF FOOD POISONING CAUSED BY FOODBORNE BACTERIA**

(71) Applicant: SkyLab AG, 1066 Epalinges (CH)
(72) Inventor: Belous, Elena Yur'evna, 121309 MOSCOW (RU); Filatov, Viktor Andreevich, 119415 MOSCOW (RU); Evseev, Grigory Gennadievich, 142803 STUPINO, MOSCOW REGION (RU)
(74) Representative: Glawe, Delfs, Moll

(57) **Abstract**

The invention relates to a cleaning composition comprising at least one oxygen bleaching agent, at least one protease, at least one manganese compound and at least one cyclodextrin and the use of said composition in hand and automatic machine dishwashing.

## Description

### FIELD OF THE INVENTION

The invention is related to the field of detergents and consists in biodegradable washing quadrotechnology based on oxygen bleaching agent, oxidant-stable protease, manganese compounds, and modified beta-cyclodextrin for hygienic cleansing of dishware and dishwashing machines at lower temperatures, and for prevention of food poisoning caused by bacteria *Salmonella enterica, Escherichia coli, Enterococcus faecalis, Listeria monocytogenes, Klebsiella pneumonia, Bacillus cereus* and *Staphylococcus aureus.* The invention is related to quadrotechnology and the use thereof in the compositions of dry, granular, and liquid dishwashing detergents, and comprises the components A, B, C, and D, wherein:
(A) An oxygen bleaching agent, in particular, sodium percarbonate;
(B) Biotechnology-derived protease, which is stable in the presence of oxidants and metal salts, in particular, subtilisin;
(C) Manganese compounds, in particular, organic manganese salt complexes, at oxidation states +2 or +3;
(D) Cyclodextrins of plant origin, in particular, beta-cyclodextrins and modifications thereof,
wherein the component ratio by weight with reference to active ingredients is (15-25):(0.01-0.5):(0.001-0.1):(0.005-0.1)% wt, respectively.

The composition is intended for use in the household chemicals for automated dishwashing, which allow to improve the efficacy of hygienic cleansing and removal of foodborne bacteria in dishwashers operating in eco-modes, short cycles and at lower dishwashing temperatures. The composition in biodegradable, safe for dishwashing machine and can be used for preparation of the household chemicals, such as dry, granular, and liquid stabilized detergents or concentrates for automated dishwashing, cleansing of kitchen utensils and surfaces, and thus providing sustained cleanliness. The use of the composition contributes to considerable risk reduction of food poisoning and nutritional disorders in consumers, caused by specific effects of resistant foodborne bacteria related to the causative agents of food toxic infections.

### BACKGROUND OF THE INVENTION

Dishwashers are becoming increasingly attractive for the consumers because this household appliance provides comfortable and effective method of cleansing the dishes and kitchen utensils from contamination and food. In European countries, Turkey, USA, Canada and MEA region countries more than 65% of consumers have dishwashing machines in their households [Euromonitor; Electrolux Market Overview 2018 for Global figure]. As estimated by the analysts, by 2028 the dishwasher market will reach 35.08 billion USD [https://www.globenewswire.com/en/news-release/2023/04/26/2655021/28124/en/Dishwasher-Global-Market-Report-2023-Freestanding-Dishwashers-Have-Hegemony-in-the-Market-Due-to-Their-Affordability-Convenience-and-Comparable-Functionality-to-Built-In-Models.html]. Dishwashers save time and efforts because it is difficult to find time for manual dishwashing under conditions of busy schedule and modern way of life. Dishwashers save time due to washing a large amount of dishware per one washing cycle thus making them the perfect choice for large families and for the parties at home. In addition, the dishwashers are water- and energy-efficient, environmental-friendly and often equipped with energy-saving functions, such as eco-modes. Further, the dishwashers offer hygienic effect due to the use of high temperatures during the washing cycle for killing the bacteria, and this is important for the families with young children, elderly or immunocompromised persons.

The eco-mode of washing operation becomes a standard one and increasingly popular, especially among European consumers [Novozymes ADW market overview and trends, 2021]. The consumers even more often use short programs in attempt to save time, electrical energy and water consumption. For instance, environmental friendliness of detergent composition and water saving are important for 57% of European consumers [Novozymes 2021 consumer study across USA, France, Germany, Sweden, UK, Italy; N = 6000]. According to the survey among the European consumers, the following washing modes are most frequently chosen: 15.3% - short washing cycles for up to 1 hour; 22.7% - eco-mode with reduced water consumption and temperature below 50° C; 2.7% - the modes at lower temperature 40° C; 44,9% - "Automatic/default", which is a modified eco-mode. Only 9.9% European consumers use intensive washing cycle at higher temperature 65-70° C [Novozymes 2021 consumer study across USA, France, Germany, Sweden, UK, Italy; N = 6000]. Despite obvious advantages of using the eco-modes and the modes at higher temperatures, there is a principal trouble directly impacting the consumer health - low quality of removal of foodborne bacteria by short cycles at low temperatures.

Presently compressed tablets are the most common detergent form for the dishwashers. In 2022 the global market value of dishwasher tablets reached 567.5 million US dollars. According to expectations of IMARC Group, the global market value will demonstrate compound annual growth rate (CAGR) at 6.2% during 2023-2028, and will reach 832.8 million US dollars by 2028 [https://www.imarcgroup.com/dishwasher-tablets-market]. It appeared that 87% of consumers globally prefer tablets/capsules largely because of effective cleansing and convenience [Novozymes 2021 consumer study across USA, France, Germany, Sweden, UK, Italy; N = 6000]. The dishwasher tablets are classified as the detergent used for dishware cleansing and washing in the dishwasher. Such tablets comprise active ingredients (polymers, chelating agents) for hard water softening, oxygen bleaching agents removing stubborn spots, enzymes for quick dissolution of carbohydrate, protein and fatty stains, and surfactants for cleansing and making the dishware bright after washing. Over the years the dishwasher tablets became popular as the most convenient and effective alternative to conventional powder detergents, because they minimize the losses, reduce the spillage risk, and protect the dishwashers thus extending the service life thereof.

The use of dishwashers also enables to reduce the risk of pathogenic bacteria dissemination, because a large number or potential foodborne pathogens inhabit dishware, kitchen utensils including cutting boards, and drainpipes [International project HomeBiome Project 2014]. Dishwashers offer an advantage over manual washing because the foodborne pathogens inhabit the sponges and washcloths used for dish washing. For instance, the washing sponges bear *Acinetobacter* at high levels, which can cause serious infections and contribute to wound or burn contamination, and *Moraxella* spp. clumps, which are more often associated with bad odors, and other strains of pathogenic microorganisms. Reported density of bacteria reached 54 billion bacterial cells per each cm of sponge [Cardinale, M., Kaiser, D., Lueders, T. et al. Microbiome analysis and confocal microscopy of used kitchen sponges reveal massive colonization by Acinetobacter, Moraxella and Chryseobacterium species. Sci Rep 2017, 7, 5791].

Since the foodborne pathogens inhabit the households, the regulation of household microbiome becomes the key area in development of innovative detergents. The household microbiome means a totality of microorganisms inhabiting inner surfaces of the house. Said surfaces include all rooms of humans, home appliances, table tops, floor, windows and glasses, water sources and all kitchen utensils. The household microbiome can have considerable impact on human health, because it can contribute to food-borne diseases. Food-borne diseases also called food poisoning are caused by consumption of contaminated food, contaminated water, poor hygiene and inappropriate cleansing of domestic goods. Food-borne diseases can be caused by various microorganisms including bacteria, fungi, viruses, and parasites. Foodborne diseases have considerable impact on global health and economy. Based on WHO estimates, globally about 600 million cases of food-borne diseases are recorded annually including 420 000 cases leading to the death [WHO Statistical Statement, 2021]. Annually one million of food poisoning cases are recorded only in Great Britain, among them 20% of cases are associated with hospitalization and severe consequences [Statistics of Food Standards Agency]. Similar situation is known in France, where 750 million cases of food poisoning are recorded, including 15% of cases associated with hospitalization and severe health consequences [Statistics of Food Standards Agency].

The analysts estimate the annual impact of food-borne diseases global economy at the level of 110 billion USD [BOS, 2015]. These include direct expenditures on medical treatment, lost productivity, and early death. The symptoms of food-borne diseases can vary depending on causative microorganism. General symptoms include diarrhea often bloody diarrhea, severe abdominal pain and fever. In severe cases or in the event of compromised immunity the foodborne diseases can also lead to dehydration, renal failure and even death of the person.

Ingestion of contaminated fruit and vegetables is one of the leading causes of foodborne diseases. Contamination may occur in the course of manufacture, transportation or storage, and can be caused by a number of factors, such as poor hygiene practice or use of contaminated water. The studies showed that *Escherichia coli, Salmonella* and *Listeria monocytogenes* are detected on the surface of fruit, vegetable, kitchen utensils and dishware [Guo et al., 2015].

Consumption of contaminated meat and poultry can be another common cause of developing food-borne diseases. These products can be contaminated in the course of manufacture, transportation or storage. The most common pathogens are Salmonella, Campylobacter and Listeria monocytogenes [EFSA, 2021].

Ingestion and use of contaminated water are also a serious cause of food-borne diseases. The water contamination can be caused by a number of factors including inadequate sanitary conditions, contamination from sewage system or livestock wastes. Common water-borne pathogens include *Escherichia coli, Salmonella* and *Campylobacter,* and also viruses, such as norovirus and hepatitis A viruses. Tap water is used in the automated dishwashers, therefore, the cycles with low water temperature significantly increase the risk of inadequate cleansing and retaining of food spoilage bacteria of the dishware surface.

In spite of the fact that dishwasher is more efficient with respect to cleansing the kitchen utensils compared to manual dish washing, the food infection challenges are still relevant. A dishwasher is unable to handle the complex stain especially when heavily loaded. The pathogenic bacteria can also penetrate and grow in the cracks in wooden or plastic dishes, while to clean such cracks at lower temperature is virtually unmanageable task. Many food-borne pathogens are resistant to cold water, and the washing modes at higher temperatures are required. Thus, the pathogenic microbiome fraction should be regarded as important factor while developing innovative dishwasher detergents exhibiting marked hygienic effect towards resistant foodborne bacteria.

Main food disorder agents and the effects thereof on the human health are discussed below. The emphasis is placed on nine bacteria, which can cause food poisoning in humans at home: *Staphylococcus aureus, Escherichia coli, Pseudomonas aeruginosa, Enterococcus faecalis, Klebsiella pneumonia, Salmonella enterica, Bacillus cereus, Listeria monocytogenes* and *Serratia marcescens.*

*Staphylococcus aureus* (*S. aureus*), including MRSA strains, is among the most common food spoilage and food poisonings causative agents, which is resistant to high washing temperature, heat processing and antibiotics. Poor hygiene and inadequately rare home cleaning leads to growth of *S. aureus* [Carmo et al., 2018]. MRSA was found in 97% of households and was isolated from all kitchen surfaces and all houses, including kitchen and bathroom water-bowls, table tops, faucet knobs, kitchen drain valves, washing sponges and cloths, kitchen towel, and pet bowls. The entrance of *S. aureus* into gastrointestinal tract through the contact with hands or unwashed dishware leads to diarrhea, fever, gastrointestinal diseases (stomatitis, gastritis, enteritis, colitis, cholecystitis, gastroenterocolitis) and other systemic diseases of the body organs.

*Escherichia coli* (*E. coli*) is a Gram-negative bacterium, transmitted through food products, pets, and kitchen utensils, which were not adequately heat-treated [Adiga I, L SK, Mustaffa MB, Bismi NB, Yusof NB, Ibrahim NB, et al. Bacterial Contamination in the Kitchen: Could It Be Pathogenic? WebmedCentral MEDICAL EDUCATION 2012;3(4):WMC003256]. These bacteria are resistant to high washing temperature and to the majority of detergents [Khaneja et al. 2019; Martin et al. 2021]. The entrance of *E. coli* into gastrointestinal tract through the contact with hands or unwashed dishware leads to diarrhea, colitis, meningitis, sepsis, and urinary tract infection.

*Pseudomonas aeruginosa* (*P. aeruginosa*) is a Gram-negative bacterium inhabiting wet areas, kitchen surfaces, fruit and vegetables; therefore it can be transmitted through consumption of unwashed products and from kitchen utensils [https://www.webmd.com/a-to-z-guides/pseudomonas-infection]. *P. aeruginosa* is highly resistant to many antibiotics and can survive in cold water up to several weeks. *P. aeruginosa* is a causative agent of nosocomial infections due to the compromised immunity and resistance to numerous commercially available antibiotics [Goossens H. Susceptibility of multi-drug-resistant Pseudomonas aeruginosa in intensive care units: results from the European MYSTIC study group // Clin Microbiol Infect. - 2003. - 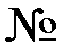 9. - pp. 980-983].

*Enterococcus faecalis* (*E. faecalis*) is a Gram-negative bacterium surviving in various conditions and on different surfaces: kitchen tops, dishware, kitchen utensils, food products under poor washing hygienic practices. *E. faecalis* is resistant to exposure of most detergents [Zhang et al. 2018], and can survive in cold water. *E. faecalis* is a causative agent of nosocomial infections due to the compromised immunity and resistance to numerous commercially available antibiotics [Ryan KJ, Ray CG, eds. (2004). Sherris Medical Microbiology (4th ed.). McGraw Hill. pp. 294-295].

*Klebsiella pneumonia* (*K. pneumonia*) is a Gram-negative bacterium, which is highly resistant to antibiotics and surface washing [Paczosa M. K., Mecsas J. Klebsiella pneumoniae: Going on the Offense with a Strong Defense // Microbiology and molecular biology reviews: MMBR. - 2016. - Vol. 80. no. 3 (September). - P. 629-661]. This bacterial species can be detected in more than 20% of European households [Adiga I, L SK, Mustaffa MB, Bismi NB, Yusof NB, Ibrahim NB, et al. Bacterial Contamination in the Kitchen: Could It Be Pathogenic? WebmedCentral MEDICAL EDUCATION 2012;3(4):WMC003256]. *K*. *pneumonia* forms biofilms, and thus can remain on the surfaces even under high temperature exposure and washing with cold water. These bacteria can be eliminated by most detergents [Ganesan et al. 2020]. The ingestion of *K. pneumonia* leads to nosocomial infections, pneumonia, sepsis, urinary tract infections, bacteremia, meningitis, and liver disorders.

*Salmonella enterica* (*S. enterica*) is a Gram-negative bacterium, which can grow in the most extreme environments, and is the most common bacteria causing food poisoning globally. *S. enterica* is a leading food-borne pathogen in USA and European countries, which is responsible for the most deaths and associated with the highest cost burden [Batz MB, Hoffmann S, Morris JG (July 2012). "Ranking the disease burden of 14 pathogens in food sources in the United States using attribution data from outbreak investigations and expert elicitation". Journal of Food Protection. 75 (7): 1278-1291]. *S. enterica* is resistant to the most antibiotics, heat processing, and high temperature dishwasher cycles even at 75°C [Rahkila R, Sivela S, Korkeala H., 2010]. Food products, kitchen utensils, pets, dirt, and soil are the most important routes of infection [Andino A, Hanning I (2015-01-13). "Salmonella enterica: survival, colonization, and virulence differences among serovars". The Scientific World Journal. 2015: 520179]. The entrance of *S. enterica* into gastrointestinal tract through the contact with hands or unwashed dishware results in diarrhea, fever, spasms and colic, vomiting and severe dehydration [WHO Reports «Salmonella (non-typhoidal)», December 2016].

*Bacillus cereus* (*B. cereus*) is a Gram-positive bacterium forming stable spores in soil, on food products and kitchen utensils [Tumbull PC (1996). "Bacillus". In Baron S, et al. (eds.). Baron's Medical Microbiology (4th ed.). University of Texas Medical Branch]. *B. cereus* is resistant to cold water washing and to conventional detergents [Notermans S, Dufrenne J, Te Giffel M., 1997]. The entrance of B. cereus into gastrointestinal tract through the contact with hands or unwashed dishware results in nausea, diarrhea, and food toxicoinfections [Kotiranta A, Lounatmaa K, Haapasalo M (February 2000). "Epidemiology and pathogenesis of Bacillus cereus infections". Microbes and Infection. 2 (2): 189-198].

*Listeria monocytogenes* (*L. monocytogenes*) is a Gram-positive bacterium causing serious food infections in pregnant women and immunocompromised persons. In contrast to the other bacteria, *L. monocytogenes* is an intracellular parasite, which is responsible for 20-30% of fatal outcomes of food-borne diseases listeriosis [Pizarro-Cerda J, Cossart, P (2019). "Microbe Profile: Listeria monocytogenes: a paradigm among intracellular bacterial pathogens". Microbiology. 165 (7): 719-721]. In EU a 16% annual increase of listeriosis is observed. In EU countries and USA the mortality rate due to listeriosis is higher that mortality rate resulting from food poisoning due to *Salmonella spp.* and *Clostridium botulinum* ["The European Union summary report on trends and sources of zoonoses, zoonotic agents and foodborne outbreaks in 2014". EFSA Journal. 13 (12) 2015]. *L. monocytogenes* forms superficial biofilms making washing with conventionally used detergents, disinfectants and hot water at temperatures below 75°C inefficient [Rahkila R, Sivela S, Korkeala H., 2010; Alakomi HL, Henell E, Tapiainen T, Richardson M, Ahvenainen R., 2010].

Many of the listed bacteria belong to ESKAPE group, i.e. they are key highly virulent and antibiotic-resistant bacterial pathogens. WHO emphasizes the need for developing new approaches to infection control and prevention of antibiotic resistance [https://www.who.int/ru/news-room/fact-sheets/detail/antibiotic-resistance]. Today 700 thousand people on the planet die annually because of resistance to antibacterials, and, according to expert estimations, in developed countries the number of deaths associated with antibiotic resistance can increase to 10 million people by 2050. This stresses the need for development the innovative household chemicals, including dishwasher tablets, which comprise the ingredients exhibiting antibacterial activity without causing antibiotic resistance.

The challenge of elimination of foodborne bacteria by cold water or conventional detergents is one of the most actual presently, because the majority of people do not use special products for washing the dishware, vegetables and fruit, and use water at the temperature 20-40 °C. Existing commercially available dishwasher tablets can comprise antibacterial ingredients: quaternary ammonium compounds, surfactants, sodium hypochlorite, benzotriazoles, polycarboxylates, sodium percarbonate, sodium carbonate (soda).

Quaternary ammonium compounds (QACs) are permanently charged molecules regardless of pH of the solution. Cations of quaternary ammonium are used for household purposes, for instance, as antimicrobial agents. In addition, QACs exhibit antiviral activity against SARS-CoV-2, for this reason the use thereof increased during COVID-19 epidemy. However, these compounds have negative effects on humans and on environment. Adverse environmental impacts include acute and chronic toxicity for susceptible aquatic organisms, non-biodegradability as follows from certification by Ecolabel EU, and antibiotic resistance. Suspected or known adverse health implications include skin and respiratory effects, reproductive toxicity, metabolic disturbance, and disturbance of mitochondrial function upon skin contact or ingestion. Furthermore, QAC role in development of antimicrobial resistance was also demonstrated [William A. Arnold, et al. Environmental Science & Technology 2023].

Surfactants, isopropyl alcohols, triclosan and chlorohexidine are the most commonly used additives to dishwashing detergents. The studies showed that not all dishwashing detergents comprising the above additives ae able to efficiently destroy four bacteria (*Pseudomonas aeruginosa, Escherichia coli, Staphylococcus aureus* and *Enterococcus faecalis*)*.* For instance, chlorohexidine and triclosan demonstrated good efficacy against all four bacteria. However, despite proved efficacy chlorohexidine and triclosan are associated with antibiotic resistance and have low degradability potential in environment [Halden, Rolf U et al. The Florence Statement on Triclosan and Triclocarban. Environmental health perspectives vol. 125,6 064501. 20 Jun. 2017, doi:10.1289/EHP1788; http://copublications.greenfacts.org/en/triclosan/index.htm1. The use of chlorohexidine induce resistance to colistin, which is a last-line reserve antibiotic [Hashemi Marjan M. et al. Proteomic Analysis of Resistance of Gram-Negative Bacteria to Chlorhexidine and Impacts on Susceptibility to Colistin, Antimicrobial Peptides, and Ceragenins // Frontiers in Microbiology. - Vol. 10. - 2019]. Daily use of QAC, triclosan, chlorohexidine leads to considerably increased antibiotic resistance of foodborne bacteria and is detrimental for environment.

Sodium hypochlorite is a widely known chlorine bleaching agent, an inorganic chemical compound of formula NaOCl. Anhydrous sodium hypochlorite is very unstable and prone to explosive degradation upon heating or friction [Urben P (2006). Bretherick's Handbook of Reactive Chemical Hazards. Vol. 1 (7th ed.). Elsevier. p. 1433]. The compound is readily degradable at room temperature and due to instability, the use of sodium hypochlorite in the household chemicals is rather limited. Taking into consideration the dishwasher tablet format the instability of hypochlorite crystals drastically hampers the use of the compound in such products.

Benzotriazoles (BTS) act as preservatives. These compounds are virtually water insoluble, thus they exhibit limited antibacterial activity during the washing cycle in dishwasher. After the washing cycle in dishwasher 99% of benzotriazole is discharged with waste water [Hussein Janna et al. From Dishwasher to Tap? Xenobiotic Substances Benzotriazole and Tolyltriazole in the Environment. // Environmental Science & Technology 2011 45 (9), 3858-3864], and 30% of the compound does not degrade [Vetter, W., Lorenz, J. Determination of benzotriazoles in dishwasher tabs from Germany and estimation of the discharge into German waters. Environ Sci Pollut Res 2013, 20. 4435-4440]. According to estimations by scientists, in Germany about 80 tons of BTS are discharged annually into the water-supply system. Chinese scientists demonstrated that benzotriazole can exhibit cardiotoxicity upon ingestion [Yang, Chun et al. "Pollution characteristics, exposure assessment and potential cardiotoxicities of PM2.5-bound benzotriazole and its derivatives in typical Chinese cities." The Science of the total environment vol. 809 (2022): 151132].

Sodium percarbonate, or sodium carbonate peroxide is sodium carbonate (soda) and hydrogen peroxide adduct. Sodium percarbonate is a colorless crystalline substance, hygroscopic and water-soluble [Craig W. Jones (1999). Applications of hydrogen peroxide and its derivatives. Royal Society of Chemistry. ISBN 0-85404-536-8]. Sodium percarbonate is used in dishwasher tablets to remove colored stains. In dishwasher tablets the bleaching properties of sodium percarbonate are implemented through peracetic acid formation only at temperatures above 60°C. Peracetic acid exhibits antibacterial activity at temperatures 40-60°C and pH 3.0-7.5 [Dibo Liu et al. Antibacterial effects of peracetic acid disinfection assessed by culturability, enzyme activity and flow cytometry // Aquaculture, Vol. 565, 2023, 739138], while at the other conditions the level of peracetic acid formation is considerably lower [https://orca.cardiff.ac.uk/id/eprint/141503/]. Thus, it is challenging to use sodium percarbonate in dishwashers. Peracetic acid is not released at low temperatures, eco-modes and short cycles, while at high temperature (>40°C) released peracetic acid will not exhibit antibacterial effect. To have antibacterial effect the catalyst is needed in order to enhance peracetic acid formation, and specific pH range [Betz, M. and Cerny, G.. "Antimicrobial effects of bleaching agents" Tenside Surfactants Detergents, vol. 37, no. 4, 2000. 230-235].

Manganese compounds in the composition of dishwasher tablets can act as antibacterial agent, because manganese oxides and ions contribute to formation of reactive oxygen species and oxidative stress in bacteria [Li M, Li G, Wang H, Yuan L. The chemodynamic antibacterial effect of MnOx nanosheet decorated silicon nanowire arrays. Mater Adv. 2022;3(1):526-533]. Manganese compounds can also serve as bleaching catalysts [Ronald Hage, Johannes W. de Boer, Fabien Gaulard, Karin Maaijen, Chapter Three - Manganese and Iron Bleaching and Oxidation Catalysts, Editor(s): Rudi van Eldik, Colin D. Hubbard, Advances in Inorganic Chemistry, Academic Press, Volume 65, 2013, Pages 85-116, ISSN 0898-8838]. However, organic manganese compounds exhibit very low stability and induce sodium percarbonate degradation even in the presence of small amount of water [Tomlinson A., Carnali J. A review of key ingredients used in past and present autodishwashing formulations and the physicchemical processes they facilitate // Handbook for cleaning/decontamination of surface], and for this reason the use of said compounds in dry or tableted autodishwashing formulations under prolonged storage is considerably limited. Mangenese salts readily form manganese oxides with transitional oxidation number, which are very reactive and destabilize organic compounds with alkyl radicals and under radiation [Risch M., Kelsey A., Han S.B., Regier T.Z., Peak D., Sayed S.Y., Wei C., Xu Z., Shao-Horn Y. Redox Processes of Manganese Oxide in Catalyzing Oxygen Evolution and Reduction: An in Situ Soft X-ray Absorption Spectroscopy Study, The Journal of Physical Chemistry C 2017 121 (33), 17682-17692]. Manganese salts, which are readily oxidized to compound manganese oxides, are noncompatible with hydrogen peroxide, organic compounds because of selective catalytical oxidation [Ghosh SK. Diversity in the Family of Manganese Oxides at the Nanoscale: From Fundamentals to Applications. ACS Omega. 2020 Oct 5;5(40):25493-25504]. Because of these features the manganese compounds are to be used in special protected forms, capsulated complexes intended for sodium percarbonate preservation, while such forms are not available at the household chemical market and is under investigated by the experts in household chemistry.

The tablet composition often comprises subtilisin - proteolytic enzyme falling within serine proteinase group. Subtilisin is produced by bacteria predominantly of *Bacillus* genus, in particular, by *Bacillus subtilis,* for protection against other microorganisms. Subtilisin is involved in proteinous stain cleavage through peptide bond hydrolysis. The enzyme exhibits selective antimicrobial properties, i.e. it has limited activity spectrum covering only Gram-positive microflora [Rachanamol, R.; Lipton, A.; Thankamani, V.; Sarika, A.; Selvin, J., 2017]. Subtilisin is widely used in the household chemicals [Veluchamy et al., 2011]. However, in dishwasher tablets subtilisin proteolytic activity is considerably complicated, because subtilisin as the majority alkaline proteases are very vulnerable to bleaching agents, and are destroyed by bleaching agents at various temperatures [Tarek, H.; Nam, K.B.; Kim, Y.K.; Suchi, S.A.; Yoo, J.C. Biochemical Characterization and Application of a Detergent Stable, Antimicrobial and Antibiofilm Potential Protease from Bacillus siamensis. Int. J. Mol. Sci. 2023, 24, 5774]. The presence of percarbonate, which is an ingredient of the composition and also a bleaching agent, is detrimental for activities of enzymes, in particular, of subtilisin protease [Ljubica Vojcic et al., Advances in protease engineering for laundry detergents, New Biotechnology, Volume 32, Issue 6, 2015, Pages 629-634, ISSN 1871-6784]. Importantly, subtilisin stability and especially thermal stability are determined by the presence of disulfide bridges in the protein structure [Ljubica Vojcic et al, Advances in protease engineering for laundry detergents, New Biotechnology, Volume 32, Issue 6, 2015, Pages 629-634, ISSN 1871-6784]. The combination of manganese compounds and percarbonate leads to reactive oxygen species production, which contribute to oxidation and disruption of disulfide bonds [Shuwen Jianget al., Oxidation of protein disulfide bonds by singlet oxygen gives rise to glutathionylated proteins, Redox Biology, Volume 38, 2021, 101822, ISSN 2213-2317] in subtilisin structure, thus leading to virtually complete loss of subtilisin activity and stability. Manganese compounds in the presence of sodium percarbonate or hydrogen peroxide form manganese oxide-based oxidants, which induce organic bonds disruption without further restoration, and reduce the bleaching potential of sodium percarbonate [Ghosh SK. Diversity in the Family of Manganese Oxides at the Nanoscale: From Fundamentals to Applications. ACS Omega. 2020 Oct 5;5(40):25493-25504]. This fact imposes considerable limitation on combined use of percarbonate, manganese compounds and subtilisin protease in the composition of autodishwashing formulations at any temperature.

Despite the conflict between subtilisin, sodium percarbonate and manganese compounds described in literature, the authors of invention unexpectedly found a synergistic effect between sodium percarbonate, manganese compounds, protease, and beta-cyclodextrins due to stabilization of compounds, when present together, and enhancement of their activity against resistant foodborne bacteria. When all components were combined in the composition of dishwasher tablets, high antibacterial activity against *Salmonella enterica, Escherichia coli, Enterococcus faecalis, Listeria monocytogenes, Bacillus cereus* and *Staphylococcus aureus* was observed at low temperature washing cycles, under conditions, previously associated with bacterial resistance. It was the combination of the four components that provided synergistic effect and stability, and enabled to resolve critical physical-chemical incompatibilities.

In several patent documents or commercial products both components are implemented either in combination or separately, however, the proposed composition and technical result thereof is not mentioned, or the use of said ingredients either in combination or separately addresses other technical tasks. For instance, in the document WO2022200053 of 10.03.2022 combined use of sodium percarbonate and manganese bleaching catalyst in powder detergent composition for autodishwashing is reported. Despite the use in the composition of household chemicals, the authors of said document emphasize the use of composition for bleaching and stain removing (in particular, tea and coffee spots), without mentioning antibacterial effect against resistant foodborne bacteria, and stabilization of oxygen agent under high humidity conditions. Furthermore, in the document benzotriazole is listed as an ingredient of composition, which, as was above mentioned, when used in dishwashing formulations, persists in large amounts in waste waters and water basins characterized by low biodegradability. In addition, the temperature ranges are not considered in the document, 40°C is the only mentioned temperature, which is, probably, required to dissolve the capsule shell. Therefore, the efficacy of composition based on sodium percarbonate and manganese bleaching catalyst at lower (20-40°C) and at higher temperatures of autodishwashing operation is not supported.

In the document WO2021158429 of 04.02.2020 combined use of sodium percarbonate, manganese bleaching catalyst and proteases in powder or liquid detergent composition for autodishwashing is reported. Despite the use in the field of household chemistry, the authors of said document emphasize the use of composition for bleaching and stain removing (in particular, tea and coffee spots), without mentioning synergistic antibacterial effect against resistant foodborne bacteria at less intensive dishwashing cycles, and stabilization of oxygen agent under high humidity conditions. The composition does not comprise cyclodextrins, contributing to stabilization of manganese compounds in the presence of sodium percarbonate and protease. In this document the use of chelating agents is disclosed, while such agents can adversely affect the protease activity because the proteases use alkaline earth metal ions as cofactors. In addition, in the document benzotriazole in listed as a component of composition, which, as was above mentioned, when used in dishwashing formulations, reaches the water basins in large amounts. The Application does not cover wide temperature ranges and washing modes at lower temperatures (20-40°C), 45°C is the only mentioned temperature. Therefore, no conclusion can be made on efficacy of composition components at lower (20-30°C) temperatures in respect to the claimed effects.

In the document EP2220205 of 05.11.2008 combined use of sodium percarbonate and manganese bleaching catalyst in powder detergent composition for dishwashers. Despite the use in the field of household chemicals, the authors of said document emphasize the use of composition for bleaching and stain removing (in particular, tea and coffee spots), without mentioning synergistic antibacterial effect against resistant foodborne bacteria, and stabilization of oxygen agent under high humidity conditions. The composition does not comprise cyclodextrins, contributing to stabilization of manganese compounds in the presence of sodium percarbonate and protease. The document does not consider claimed temperature ranges and washing cycles at lower temperatures (20-40°C), 50-55°C is the only mentioned temperature range. Therefore, no conclusion can be made on efficacy of composition components at lower (20-30°C) temperatures in respect to the claimed effects. In addition, the authors do not indicate preferable pH of the composition, so no grounds are given for judgement on stability or efficacy of the proposed composition.

Document WO2005112647 of 21.05.2004 discloses antimicrobial composition comprising a peroxide donor and acid in combination with organometallic manganese catalyst. The composition does not comprise cyclodextrins, contributing to stabilization of manganese compounds in the presence of sodium percarbonate and protease and potentiating antibacterial effect at lower temperatures and within short 20-minute period. Antibacterial activity was demonstrated only against *Pseudomonas aeruginosa, Escherichia coli, Staphylococcus aureus* and *Enterococcus hirae,* among them two bacteria are not the causative agent of intestinal infections. The authors of this document did not demonstrate antibacterial activity against the other bacteria, for instance, *Listeria monocytogenes, Bacillus cereus* and *Salmonella enterica,* which are the most hazardous food spoilage and causative agents related to food toxic infection. Therefore, the referred Application does not comprise sufficient information and supporting evidence on composition efficacy against causative agents of food infection when used as dishwashing formulation. Importantly, in the document the concerned variant of composition (dry or liquid form or granulate) and physical-chemical characteristics of composition (pH, temperature, mode of autodishwashing), are not disclosed, thus potential use of the data is limited.

In the document WO2019186159 of 27.03.2018 a bleaching composition is disclosed, said composition comprising sodium percarbonate and manganese bleaching catalyst. Though the authors accentuate a bleaching effect, they also mention possible antibacterial effect of the composition. The authors state that disclosed antimicrobial formulation can be used to treat skin infections or to prevent cut wound infection, etc. in the form of ointment or solution, which are not within the field of household chemicals and not intended for elimination of foodborne bacteria. Antibacterial activity was demonstrated only against *Pseudomonas aeruginosa, Escherichia coli, Staphylococcus aureus* and *Enterococcus hirae,* among them two bacteria are not the causative agent of intestinal infections. The authors of this document did not demonstrate antibacterial activity against the other bacteria, for instance, *Listeria monocytogenes, Bacillus cereus* and *Salmonella enterica,* which are the most hazardous food spoilage and food toxic infection causative agents. The evidence of composition efficacy against foodborne causative agents is insufficient, in particular in respect of using as a dishwasher detergent at particular pH (8.0-12.0) level at under particular test conditions. Importantly, the authors indicate optimal composition pH as pH 9 or lower, thus one can face difficulties when using said composition in dishwasher detergents at pH 10-12, while no supporting experiments of the invention are provided.

The technical result of innovative quadrotechnology consists in effective inhibition of growth and biofilms formation by foodborne bacteria, which are resistant to low temperatures, and preserving stability of all four components jointly present in autodishwashing detergent formulations. The composition is active in wide pH range, in particular, from pH 7.0 to pH 12.0, and in wide temperature range, in particular from +35 to +60°C, thus extending the application field in environmental friendly formulations to protect the planet resources.

Specifically, combination of four components demonstrated the synergistic effect, provided stability and allowed to resolve critical physical-chemical incompatibilities. It has been found that beta-cyclodextrins in combination with protease and manganese compounds form clathrate intramolecular complexes and enable stabilization of subtilisin structure and subtilisin protection from oxidation by manganese compounds, and also stabilization of manganese compounds from early interaction with sodium percarbonate. During automated dishwashing beta-cyclodextrin-based clathrate complex releases manganese compounds to activate percarbonate and peracetic acid under lower temperatures, this is manifested by improved removal of bleached stain and marked antibacterial effect exceeding 99.9% against *Salmonella enterica, Escherichia coli, Enterococcus faecalis, Listeria monocytogenes, Bacillus cereus* and *Staphylococcus aureus* at 30°C and even within 20 minutes of contact. Specifically, combination of four components with addition of cyclodextrins, which were never used previously in the compositions of autodishwashing formulations, enables to achieve this effect compared to separate ingredients, double and triple combinations.

Sodium percarbonate, protease (subtilisin), manganese compounds, and beta-cyclodextrins with modified chemical groups are understood to mean raw materials containing respective pure active ingredients and also technical impurities, which could form in the process of manufacturing of intended raw materials. Beta-cyclodextrins and modifications thereof are entirely new ingredients for use in the household chemical formulation, which have been never used in this field. Stabilization and preservation of active oxygen, and prolongation of shelf-life of dishwasher tablets under high humidity is the extra property. In addition, neutralization of strong-smelling aromatic substances falling under different classes of organic compounds takes place: aldehydes, ketones, terpenes, amines, indole, sulfur compounds (mercaptans), organic acids and respective esters, phenols and cresols. The distinguishing feature is that the ingredients at the claimed concentrations are active only against foodborne bacteria and do not impair the custom look of dishware made of any material. The composition does not comprise aggressive inorganic surfactants, organic solvents, occlusive film-forming agents or alcohols, quaternary ammonium bases, antibacterial additives with resistance potential, chlorine of phosphorus derivatives, therefore washing and cleansing formulations based on the claimed composition can be regularly used with no harm for dishwasher and environment. Combination of the above ingredients results in synergistic effect providing comprehensive care of various kitchen utensils with a single household chemical formulation for daily use.

### SUMMARY OF THE INVENTION

In the first aspect the invention is related to a composition for use in household chemical formulations for automated dishwashing, which is active at pH 7.0-12.0 and water hardness from 0 to 28.0° dH, comprising:
(A) An oxygen bleaching agent, in particular, sodium percarbonate;
(B) Biotechnology-derived protease, which is stable in the presence of oxidants and metal salts, in particular, subtilisin;
(C) Manganese compounds, in particular, organic manganese salt complexes, at oxidation states +2 or +3;
(D) Cyclodextrins of plant origin, in particular, beta-cyclodextrins and modifications thereof,
wherein the component ratio by weight with reference to active ingredients is (15-25):(0.01-0.5):(0.001-0.1):(0.005-0.1)% wt, respectively.

In the second aspect the invention is related to the use of composition of the invention in the household chemical formulation for automated dishwashing. A household chemical formulation of the invention can comprise 10 to 25.00% wt. of composition of the invention.

### DETAILED DESCRIPTION OF THE INVENTION

In the first aspect the invention is related to a composition for use in household chemical formulations for automated dishwashing, which is active at pH 7.0-12.0 and water hardness from 0 to 28° dH, comprising:
(A) An oxygen bleaching agent, in particular, sodium percarbonate;
(B) Biotechnology-derived protease, which is stable in the presence of oxidants and metal salts, in particular, subtilisin;
(C) Manganese compounds, in particular, organic manganese salt complexes, at oxidation states +2 or +3;
(D) Cyclodextrins of plant origin, in particular, beta-cyclodextrins and modifications thereof,
wherein the component ratio by weight with reference to active ingredients is (15-25):(0.01-0.5):(0.001-0.1):(0.005-0.1)% wt, respectively.

Weight fraction of component A in said weight ratio is 15, 20 or 25, or any values in-between.

Weight fraction of component B in said weight ratio is 0.01, 0.02, 0.03, 0.04, 0.05, 0.06, 0.07, 0.08, 0.09, 0.1, 0.15, 0.2, 0.25, 0.3, 0.35, 0.4, 0.45 or 0.5 or any values in-between.

Weight fraction of component C in said weight ratio is 0.001, 0.002, 0.003, 0.004, 0.005, 0.01, 0.02, 0.03, 0.04, 0.05, 0.06, 0.07, 0.08, 0.09 or 0.10 or any values in-between.

Weight fraction of component D in said weight ratio is 0.005, 0.006, 0.007, 0.008, 0.009, 0.01, 0.02, 0.03, 0.04, 0.05, 0.06, 0.07, 0.08, 0.09 or 0.10 or any values in-between.

A composition can be distinguished by that oxygen bleaching agent is sodium percarbonate, CAS No 15630-89-4, such as Sodium Percarbonate (P-70) or Sodium Percarbonate (Coated)/ Zhejiang Jinke Peroxides. Said products are commercially available and are intended for inclusion into the formulations.

A composition can be distinguished by that biotechnology-derived protease stable in the presence of oxidants and metal salts is a serine protease (subtilisin), which is obtained by biotechnological method from microorganisms, in particular, from bacteria. A substance or a commercially available product with CAS No 9014-01-1 can serve as a protease.

A composition can be distinguished by that manganese compounds, in particular, organic complexes of manganese salts at oxidation state +2 and +3 are organic or inorganic manganese compounds, such as WeylCat^{®} Pegasus or Blaze Exceed 100T Novozymes AS, or the other compounds. Said substances are commercially available products and are intended for inclusion into the formulations.

A composition can be distinguished by that cyclodextrins of plant origin are beta-cyclodextrins with CAS No 7585-39-9 or hydroxypropyl-beta-cyclodextrins with CAS No 128446-35-5. Said substances are commercially available products and are intended for inclusion into the formulations.

The present invention is related also to the use of composition of the invention in the household chemical formulation.

A household chemical formulation for automated dishwashing of the invention can comprise a composition of the invention in amount of 10.0-25.0% wt. For example, a household chemical formulation of the invention can comprise a composition of the invention in amount of 10, 11, 12, 13, 14, 15, 16, 17, 18, 19, 20, 21, 22, 23, 24 or 25% wt. or any values in-between.

In formulation of the invention acceptable excipients can be selected from the following categories of ingredients.

A formulation or a composition of the invention can comprise, % wt.:

| | |
|---|---|
| Sodium percarbonate | 15.0-25.0%, |
| Protease in particular, subtilisin | 0.01-0.5%, |
| Organic complex of manganese at oxidation state of +2 or +3 | 0.001-0.10%, |
| Hydroxypropyl-beta-cyclodextrins | 0.005-0.10%. |

The present invention is also related to the use of the composition of invention for hygienic cleansing of dishware and dishwashers at lower temperatures and for prevention of food poisoning caused by the bacteria *Salmonella enterica, Escherichia coli, Enterococcus faecalis, Listeria monocytogenes, Bacillus cereus* and *Staphylococcus aureus.*

The present invention is also related to high-efficient blend intended for use in household chemical formulations, said blend comprising: 1) sodium percarbonate (P-70) or Sodium Percarbonate (Coated)/ Zhejiang Jinke Peroxides; 2) protease (subtilisin) as a part of enzymatic complex Intensa Evity^{®} 145 T Novozymes AS; 3) organic manganese complex as a part of enzymatic complex Blaze Exceed 100T Novozymes AS, and 4) hydroxypropyl-beta-cyclodextrins, wherein said blend ingredients are taken at weight ratio (10-25):(0.01-0.5):(0.001-0.10):(0.005-0.1), respectively. Said products are commercially available and are intended for inclusion into the household chemical formulations. The above blend exhibits all advantages of the composition described in the present document is intended for use in the household chemical formulations, such as dishwasher tablets, dishwasher powder, dishwasher capsules.

Weight fraction of component (A) in said weight ratio is 15, 20 or 25 or any values in-between. Weight fraction of component (B) in said weight ratio is 0.01, 0.02, 0.03, 0.04, 0.05, 0.06, 0.07, 0.08, 0.09, 0.1, 0.15, 0.2, 0.25, 0.3, 0.35, 0.4, 0.45 or 0.5 or any values in-between. Weight fraction of component (C) in said weight ratio is 0.001, 0.002, 0.003, 0.004, 0.005, 0.01, 0.02, 0.03, 0.04, 0.05, 0.06, 0.07, 0.08, 0.09 or 0.10 or any values in-between. Weight fraction of component (D) in said weight ratio is 0.005, 0.01, 0.02, 0.03, 0.04, 0.05, 0.06, 0.07, 0.08, 0.09 or 0.10 or any values in-between.

The composition preferably does not comprise other active ingredients and/or excipients, such as active detergents and/or acceptable excipients, but can comprise said compounds. Such compounds may be or are the agents conventionally used in this field and well-known to those skilled in the art. Addition of said agent into the complex of the invention does not reverse the achievement of the claimed technical results but rather can improve them.

The scope of invention covers also the household chemical formulations, such as tableted detergent for automated dishwashing, powder detergent for automated dishwashing, granular detergent for automated dishwashing, encapsulated detergent for automated dishwashing.

In the present disclosure all weight ratios, weight fractions, weight percent as well as volume ratios, volume parts, and volume percent are given in relation to the formulation, agent, composition, or product according to the context.

In the household chemical formulation of the invention the acceptable excipients can be selected from the following categories of ingredients.

### Anionic surfactants:

Alkyl polyethylene glycol sulphate of general formula R¹-O(-CH₂-CH₂-O)n¹ (SO₃) n² X¹, wherein n¹ is in the range from 0 to 10 and indicates the number of polyethylene groups, R¹ is alkyl and/or alkenyl with hydrocarbon chain length from 5 to 22 carbon atoms, n² is in the range from 0 to 1 and indicates the number of sulphate groups, X¹ is a cation of alkali metal and/or alkaline-earth metal, ammonium, alkyl ammonium, alkanol ammonium, glucoammonium, basic amino acid;

Salts of higher carboxylic acids of general formula: R¹-CO₂X¹, wherein R¹ is alkyl and/or alkenyl with hydrocarbon chain length from 5 to 21 carbon atoms, and X¹ is a cation of alkali metal and/or alkaline-earth metal, ammonium, alkyl ammonium, alkanol ammonium, glucoammonium, basic amino acid;

Alkyl polyethylene glycol carboxylate of general formula: R⁵-O(-CH₂-CH₂-O-)n²CH₂-CO₂X⁵, wherein n² is in the range from 1 to 15, and indicates the number of polyethylene glycol groups, R⁵ is alkyl and/or alkenyl with hydrocarbon chain length from 6 to 22 carbon atoms, and X⁵ is a cation of alkali metal and/or alkaline-earth metal, ammonium, alkyl ammonium, alkanol ammonium, glucoammonium;

Alkyl sulphate of general formula R³-OSO₃X³, wherein R³ is alkyl and/or alkenyl with hydrocarbon chain length from 6 to 22 carbon atoms, and X³ is a cation of alkali and/or alkaline-earth metal, ammonium, alkyl ammonium, alkanol ammonium, glucoammonium;

Amide salt of higher fatty acid and methylglycine of general formula R⁴-C(O)-N(-CH₃)-CH₂-CO₂X⁴, wherein R⁴ is alkyl and/or alkenyl with hydrocarbon chain length from 5 to 21 carbon atoms, and X⁴ is a cation of alkali and/or alkaline-earth metal, ammonium, alkyl ammonium, alkanol ammonium, glucoammonium;

Alkyl polyethylene glycol carboxylate of general formula: R⁵-O(-CH₂-CH₂-O-)n²CH₂-CO₂X⁵, wherein n² is in the range from 1 to 15, and indicates the number of polyethylene glycol groups, R⁵ is alkyl and/or alkenyl with hydrocarbon chain length from 6 to 22 carbon atoms, and X⁵ is a cation of alkali and/or alkaline-earth metal, ammonium, alkyl ammonium, alkanol ammonium, glucoammonium;

Disubstituted salt of 2-sulphocarboxylic acid of general formula: R⁶-CH(-SO₃X⁶)-CO₂X⁶, wherein R⁶ is alkyl and/or alkenyl with hydrocarbon chain length from 4 to 20 carbon atoms, and X⁶ is a cation of alkali and/or alkaline-earth metal, ammonium, alkyl ammonium, alkanol ammonium, glucoammonium;

Mono- or disubstituted amide salt of higher carboxylic acid and glutamic acid of general formula: R⁷-C(O)-NH-CH(-CH₂-CH₂-CO₂X⁷)-CO₂X⁷, wherein R⁷ is alkyl and/or alkenyl with hydrocarbon chain length from 5 to 21 carbon atoms, and X⁷ is a cation of alkali and/or alkaline-earth metal, ammonium, alkyl ammonium, alkanol ammonium, glucoammonium or hydrogen;

Amide salt of higher fatty acid 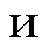 glycine of general formula: R⁸-C(O)-NH-CH₂-CO₂X⁸, wherein R⁸ is alkyl and/or alkenyl with hydrocarbon chain length from 5 to 21 carbon atoms, and X⁸ is a cation of alkali and/or alkaline-earth metal, ammonium, alkyl ammonium, alkanol ammonium, glucoammonium;

Amide salt of higher fatty acid and alanine of general formula: R⁹-C(O)-NH-CH(-CH₃)-CO₂X⁹, wherein R⁹ is alkyl and/or alkenyl with hydrocarbon chain length from 5 to 21 carbon atoms, and X⁹ is a cation of alkali and/or alkaline-earth metal, ammonium, alkyl ammonium, alkanol ammonium, glucoammonium;

Amide salt of higher fatty acid and 2-aminomethyl ethane sulphonic acid of general formula: R¹⁰-C(O)-N(-CH₃)-CH₂-CH₂-SO₃X¹⁰, wherein R¹⁰ is alkyl and/or alkenyl with hydrocarbon chain length from 5 to 21 carbon atoms, and X¹⁰ is a cation of alkali and/or alkaline-earth metal, ammonium, alkyl ammonium, alkanol ammonium, glucoammonium;

Alkylpolyglucoside hydroxypropyl sulphonate of general formula: R¹¹-O-[G]p1-O-CH₂-CH(-OH)-CH₂-SO₃X¹¹ , wherein R¹¹ is alkyl and/or alkenyl with hydrocarbon chain length from 6 to 22 carbon atoms, G is a saccharide fragment comprising 5 or 6 carbon atoms, p1 is in the range from 1 to 4, and X¹¹ is a cation of alkali and/or alkaline-earth metal, ammonium, alkyl ammonium, alkanol ammonium, glucoammonium;

Alkylpolyglucoside carboxylate of general formula: R¹²-O-[G]p2-O-CH₂-CO₂X¹², wherein R¹² is alkyl and/or alkenyl with hydrocarbon chain length from 6 to 22 carbon atoms, G is a saccharide fragment comprising 5 or 6 carbon atoms,, p2 is in the range from 1 to 4, and X¹² is a cation of alkali and/or alkaline-earth metal, ammonium, alkyl ammonium, alkanol ammonium, glucoammonium;

Amide salt of higher fatty acid and threonine of general formula: R¹³-C(O)-NH-CH(-CH(-OH)-CH₃)-CO₂X¹³, wherein R¹³ is alkyl and/or alkenyl with hydrocarbon chain length from 5 to 21 carbon atoms, and X¹³ is a cation of alkali and/or alkaline-earth metal, ammonium, alkyl ammonium, alkanol ammonium, glucoammonium;

Amide salt of higher fatty acid and amino acid obtained by protein hydrolysis of the plant raw materials, of general formula: R¹⁴-C(O)-AAX¹⁴, wherein R¹⁴ is alkyl and/or alkenyl with hydrocarbon chain length from 5 to 21 carbon atoms, AA is amino acid or peptide obtained from the plant protein hydrolysis (potential protein sources: apple, soybean, wheat, flax plant, etc.), and X¹⁴ is a cation of alkali and/or alkaline-earth metal, ammonium, alkyl ammonium, alkanol ammonium, glucoammonium.

### Amphoteric surfactants:

Disubstituted acyl amphodiacetate salt of general formula: R¹⁵-C(O)-NH-CH₂-CH₂-N(-CH₂-CO₂X¹⁵)-CH₂-CH₂-O-CH₂-CO₂X¹⁵, wherein R¹⁵ is alkyl and/or alkenyl with hydrocarbon chain length from 5 to 21 carbon atoms, and X¹⁵ is a cation of alkali and/or alkaline-earth metal, ammonium, alkyl ammonium, alkanol ammonium, glucoammonium;

Acyl amphoacetate salt of general formula: R¹⁶-C(O)-NH-CH₂-CH₂-N(-CH₂-CO₂X¹⁶)-CH₂-CH₂-OH, wherein R¹⁶ is alkyl and/or alkenyl with hydrocarbon chain length from 5 to 21 carbon atoms, and X¹⁶ is a cation of alkali and/or alkaline-earth metal, ammonium, alkyl ammonium, alkanol ammonium, glucoammonium;

Alkyl amphoacetate salt of general formula: R¹⁷-C(=N-CH₂-CH₂-N((-CH₂-CH₂-OH)-CH₂-CO₂X¹⁷)-), wherein R¹⁷ is alkyl and/or alkenyl with hydrocarbon chain length from 5 to 21 carbon atoms, and X¹⁷ is a cation of alkali and/or alkaline-earth metal, ammonium, alkyl ammonium, alkanol ammonium, glucoammonium;

Acylamidoalkyl betaine of general formula: R¹⁸-C(O)-NH-R¹⁹-N(-CH₃)₂)-CH₂-CO₂, wherein R¹⁸ is alkyl and/or alkenyl with hydrocarbon chain length from 5 to 21 carbon atoms, R¹⁹ is alkyl with hydrocarbon chain length from 1 to 4 carbon atoms;

Acylamidoalkylhydroxy sultaine of general formula: R²⁰-C(O)-NH-R²¹-N(-CH₃)₂-CH₂₋ CH(-OH)-CH₂-SO₃, wherein R²⁰ is alkyl and/or alkenyl with hydrocarbon chain length from 5 to 21 carbon atoms, R²¹ is alkyl with hydrocarbon chain length from 1 to 4 carbon atoms;

Acylamido alkylamine oxide of general formula: R²²-C(O)-NH-R²³-N(-CH₃)₂-O, wherein R²² is alkyl and/or alkenyl with hydrocarbon chain length from 5 to 21 carbon atoms, R²³ is alkyl with hydrocarbon chain length from 1 to 4 carbon atoms;

Alkylbetaine of general formula: R²⁴-N(-CH₃)₂)-CH₂-CO₂, wherein R²⁴ is alkyl and/or alkenyl with hydrocarbon chain length from 5 to 21 carbon atoms;

Alkylhydroxy sultaine of general formula: R²⁵-N(-CH₃)₂-CH₂-CH(-OH)-CH₂-SO₃, wherein R²⁵ is alkyl and/or alkenyl with hydrocarbon chain length from 6 to 22 carbon atoms;

Alkylsultaine of general formula: R²⁶-N(-CH₃)₂-CH₂-CH₂-CH₂-SO₃, wherein R²⁶ is alkyl and/or alkenyl with hydrocarbon chain length from 6 to 22 carbon atoms;

Alkylamine oxide of general formula: R²⁷-N(-CH3)2-O, wherein R²⁷ is alkyl and/or alkenyl with hydrocarbon chain length from 6 to 22 carbon atoms.

### Nonionic surfactants:

Alkylglucoside of general formula: R²⁸-O-[G]p3, wherein R²⁸ is alkyl and/or alkenyl with hydrocarbon chain length from 4 to 22 carbon atoms, G is saccharide fragment comprising 5 or 6 carbon atoms, p3 is in the range from 1 to 4;

Alkylpolyethylene glycol of general formula: R²⁹-O(-CH₂-CH₂-O-)n3H, wherein n3 is in the range from 2 to 20, and indicates the number of polyethylene glycol groups, R²⁹ is alkyl and/or alkenyl with hydrocarbon chain length from 6 to 22 carbon atoms;

Alkylpolyethylene/propylene glycol of general formula: R³⁰-O(-CH₂-CH₂-O-)n4(-CH(-CH₃)-CH₂-O-)n5H, wherein n4 is in the range from 2 to 20, and indicates the number of polyethylene glycol groups, n5 is in the range from 2 to 20, and indicates the number of polypropylene glycol groups, R³⁰ is alkyl and/or alkenyl with hydrocarbon chain length from 6 to 22 carbon atoms;

Dialkyl polyethylene glycol of general formula: R³¹-O(-CH₂-CH₂-O-)n6R³², wherein n6 is in the range from 2 to 20, and indicates the number of polyethylene glycol groups, R³¹ is alkyl and/or alkenyl with hydrocarbon chain length from 6 to 22 carbon atoms, R³² is alkyl and/or alkenyl with hydrocarbon chain length from 1 to 12 carbon atoms;

Dialkyl polethylene/propylene glycol of general formula: R³³-O(-CH₂-CH₂-O-)n7(-CH(-CH₃)-CH₂-O-)n8-R³⁴, wherein n7 is in the range from 2 to 20, and indicates the number of polyethylene glycol groups, n8 is in the range from 2 to 20, and indicates the number of polypropylene glycol groups, R³³ is alkyl and/or alkenyl with hydrocarbon chain length from 6 to 22 carbon atoms, R³⁴ is alkyl and/or alkenyl with hydrocarbon chain length from 1 to 12 carbon atoms.

### Dispersing medium for polysaccharide/solvent:

Organic alcohol of general formula: R³⁵(-OH)s1, wherein R³⁵ is alkyl with hydrocarbon chain length from 3 to 12 carbon atoms, s1 is in the range from 1 to 12, and indicates the number of hydroxylic groups located and hydrocarbon radical in random order with respect to each other;

Alkylpolypropylene glycol of general formula: H(-CH(-CH₃)-CH₂-O-)n9R³⁶, wherein n9 is in the range from 2 to 10, and indicates the number of polypropylene glycol groups, R³⁶ is alkyl with hydrocarbon chain length from 1 to 10 carbon atoms.

### pH adjusting agents:

Organic acids of general formula: R³⁷(-OH)s2(-COOH)m1, wherein R³⁷ is alkyl with hydrocarbon chain length from 1 to 12 carbon atoms, s2 is in the range from 1 to 12, and indicates the number of hydroxylic groups located and hydrocarbon radical in random order with respect to each other, m1 is in the range from 1 to 4, and indicates the number of carboxylic groups located and hydrocarbon radical in random order with respect to each other;

Solutions of alkali and/or alkaline-earth metal hydroxides, ammonium hydroxide, primary and tertiary alkyl amines, primary and tertiary alkanol amines, primary and tertiary glucamines, basic amino acids, disodium citrate, trisodium citrate.

### Chelating agent:

Trisodium methyl glycine-diacetate, tetrasodium of glutamine-diacetate, trisodium of ethylenediamine-(N,N)-disuccinate;

Phosphonic acid esters of general formula RP(O)(OR'1)n(OH)₂₋ₙ, wherein R, R' are organic radicals, in particular, alkyl, alkenyl or aryl radical; said esters may be primary (n=1, acidic phosphonates) or secondary (n=2, full phosphonate) depending on hydroxylic groups;

Organic acids, and salts formed by alkali metals, ammonium, alkyl ammonium, glucoammonium and said organic acids: citric acid, malic acid, tartaric acid, glutaric acid, adipic acid, glucuronic acid, galacturonic acid, galactaric acid, gluconic acid, phytic acid, polyitaconic acid, polyacrylic acid, polymetacrylic acid, acrylic/metacrylic and maleinic acid co-polymer, and also organic acids of general formula R38(-OH)s3(-COOH)m2, wherein R38 is alkyl with hydrocarbon chain length from 1 to 12 carbon atoms, S3 is in the range from 1 to 12, and indicates the number of hydroxylic groups located and hydrocarbon radical in random order with respect to each other, M2 is in the range from 1 to 4, and indicates the number of carboxylic groups located in hydrocarbon radical in random order with respect to each other.

### Inhibitors of stain redeposition:

Polysaccharide derivatives: sodium carboxymethyl polysaccharide, hydroxyalkyl polysaccharide, alkyl polysaccharide;

Polyvinyl pyrrolidone and the derivatives thereof; polyvinyl pyrrolidone and vinyl imidazole co-polymers;

Water-soluble salts of polyacrylic acid, polymetacrylic acid, acrylic/metacrylic and maleinic acid co-polymer.

### Anti-foaming agents:

Higher carboxylic acids of general formula: R39-CO2H, wherein R39 is alkyl and/or alkenyl with hydrocarbon chain length from 5 to 21 carbon atoms;

Higher carboxylic alcohols of general formula: R40-COH, wherein R40 is alkyl and/or alkenyl with hydrocarbon chain length from 5 to 21 carbon atoms;

Ethers of higher carboxylic alcohols of general formula: R41-O-R42, wherein R41, R42 are alkyl and/or alkenyl with hydrocarbon chain length from 4 to 22 carbon atoms;

Bis-amides of alkyldiamines and higher carboxylic acids of general formula: R43-C(O)-NH-R44-NH-C(O)-R45, wherein R43, R45 are alkyl and/or alkenyl with hydrocarbon chain length from 5 to 21 carbon atoms, and R44 is alkyl with hydrocarbon chain length from 1 to 12 carbon atoms;

### Preservatives:

Organic acids and salts of alkaline and alkaline earth metals, ammonium, alkyl ammonium, alkanol ammonium, glucoammonium of respective organic acids: benzoic acid, sorbic acid, 4-methoxybenzoic acid, salicylic acid, undecanoic acid;

Organic alcohols and phenols: phenoxy ethanol, benzyl alcohol, caprylyl glycol, ethylhexylglycerol, phenetyl alcohol, 3-methyl-4-isopropylphenol, 2,4-dichlorobenzyl alcohol;

Wide-spectrum biocidal agents: benzisothiazolinone, dodecyl propylene triamine, methylisothiazolinone;

### Fungicides: sodium pyrithione, climbazole.

Enzymes: protease (ficin, bacillolysin, subtilisin, papain, bromelain, actinidin), alphaamylase, pectate lyase, mannanase, mannosidase, cellulase, amine oxidase, feruloyl esterase, beta-glucanase, tannase, alpha-glucosidase, beta-glucosidase, alpha-galactosidase, betagalactosidase, laccase, manganese peroxidase, licheninase, xylanase, and other commercially available enzymes, which are used in laundry washing detergents, dishwashing detergents, cleaners for floors, glass, and multi-surface cleaners.

Oxygen bleaching agents: hydrogen peroxide, calcium peroxide, carbamide peroxide, ε-phthalimidoperoxycaproic acid and other commercially available ingredients.

Perfume agents containing essentials oils, as neat essential oils or as essential oils blends prepared at various proportions: orange, bergamot, lemon, lime, tangerine, grape fruit, neroli, rose wood, Yuji, lemongrass, lavender, sage, thyme, melissa, mint, tea tree, eucalypt, cedar, sandal, black pepper, pink pepper, cinnamon, cardamon, coriander, jasmine, rose, peony, cyan chamomile, or other.

### EXPERIMENTS

The examples in this specification are not considered as limiting the present invention, and are included merely for illustrative purpose and to support the achievement of the expected technical results. These examples are among many experimental findings obtained by the Authors of invention, which prove the efficacy of formulations within the scope of invention.

### Example 1.

The components intended for inclusion into composition of the invention were tested as the ingredients of various detergents for automated dishwashing. Within the scope of invention dry formulation for automated dishwashing, in particular, dishwasher tablets 10 grams were prepared (Table 1).

**Table 1. Composition of dishwasher tablets 10 grams made of the claimed composition.**

| **No** | **Ingredient** | **Content, % wt.** |
|---|---|---|
| 1 | Purified water | 0.1-2.0 |
| 2 | Caustic ash | 10-35 |
| 3 | Sodium percarbonate | 15-25 |
| 4 | Sodium carbonate | 5-10 |
| 5 | Citric acid, anhydrous | 5-10 |
| 6 | Sodium hydrocarbonate | 1-5 |
| 7 | Helating agent MGDA | 3-10 |
| 8 | PEG-4000 | 0.5-3.0 |
| 9 | Sodium polyacrylate | 5-12 |
| 10 | Acrylate-copolymer | 0.5-3.0 |
| 11 | Nonionic surfactant | 0.1-0.5 |
| 12 | Low-foaming nonionic alkoxylate-based surfactant | 1.0-4.0 |
| 13 | Hydroxypropyl-beta-cyclodextrins | 0.005-0.1 |
| 14 | Manganese salt-based oxidation catalyst (on a pure basis) | 0.001-0.10 |
| 15 | Biotechnology-derived protease (subtilisin) (on a pure basis) | 0.005-0.5 |
| 16 | Biotechnology-derived amylase (on a pure basis) | 0.001-0.05 |
| 17 | Pigments for coloring the tablet layers (total) | 0.01-0.50 |
| 18 | Perfume agents and essential oils (total) | 0.05-0.50 |
| 19 | Other excipients (if appropriate) | 0.001-3.0 |

Obtained dry tableted formulation for automated dishwashing provides highly efficient cleansing and removal of clotted stain, tea and coffee spots, neutralizes unpleasant odors, makes the dishes made from different functional materials bright and shiny, extends the service life of dishwasher, provides hygienic cleansing and eliminates foodborne bacteria in dishwashers operating in eco-modes, short cycles at any hardness of tap water within 0-28 °dH and at any pH of formulation in the range from 7.0 to 12.0. The formulation is dissolved within 8 to 9 minutes at short dishwashing cycles and leaves the dishwasher elements free from streaks, undissolved particles and deposit.

### Example 2.

The components intended for inclusion into composition of the invention were tested as the ingredients of various detergents for automated dishwashing. Within the scope of invention dry formulation for automated dishwashing, in particular, dishwasher tablets 16 grams were prepared (Table 2).

**Table 2. Composition of dishwasher tablets 16 grams made of the claimed composition.**

| **No** | **Ingredient** | **Content, % wt.** |
|---|---|---|
| 1 | Purified water | 0.1-2.0 |
| 2 | Caustic ash | 10-35 |
| 3 | Sodium percarbonate | 15-25 |
| 4 | Sodium carbonate | 5-10 |
| 5 | Citric acid, anhydrous | 3-10 |
| 6 | Sodium hydrocarbonate | 1-5 |
| 7 | Helating agent MGDA | 3-10 |
| 8 | PEG-4000 | 0.5-3.0 |
| 9 | Sodium gluconate | 10-20 |
| 10 | TAED activator | 0.5-5.0 |
| 11 | Sodium polyacrylate | 5-12 |
| 12 | Acrylate-copolymer | 0.5-3.0 |
| 13 | Polycarboxylates | 3.0-6.0 |
| 14 | Low-foaming nonionic alkoxylate-based surfactant | 1.0-4.0 |
| 15 | Hydroxypropyl-beta-cyclodextrins | 0.005-0.1 |
| 16 | Manganese salt-based oxidation catalyst (on a pure basis) | 0.001-0.10 |
| 17 | Biotechnology-derived protease (subtilisin) (on a pure basis) | 0.01-0.5 |
| 18 | Biotechnology-derived amylase (on a pure basis) | 0.001-0.05 |
| 19 | Pigments for coloring the tablet layers (total) | 0.01-0.50 |
| 20 | Perfume agents and essential oils (total) | 0.05-0.50 |
| 21 | Other excipients (if appropriate) | 0.001-3.0 |

Obtained dry tableted formulation for automated dishwashing provides highly efficient cleansing and removal of clotted stain, tea, coffee and vine spots, neutralizes unpleasant odors, makes the dishes made from different functional materials bright and shiny, extends the service life of dishwasher, provides hygienic cleansing and eliminates foodborne bacteria in dishwashers operating in eco-modes, short cycles, at lower autodishwashing temperatures, at any hardness of tap water within 0-28° dH and at any pH of formulation in the range from 7.0 to 12.0. The formulation is dissolved within 8 to 9 minutes in eco-modes of dishwashing and leaves the dishwasher elements free from streaks, undissolved particles and deposit. The formulation comprises the ingredients acting as rinse and salt, and thus is multifunctional.

### Example 3.

The composition of the invention was studied in various dishwasher tablets to evaluate the functional effects, hygienic cleansing, and elimination of foodborne bacteria (*Salmonella enterica, Escherichia coli, Enterococcus faecalis, Listeria monocytogenes, Bacillus cereus, Klebsiella pneumonia, Staphylococcus aureus*) with the use of eco-modes, short cycles and at lower temperature of dishware washing in dishwashers. Dry formulations for automated dishwashing (formulations No 1, 2, 3, 4) were prepared, in particular, dishwasher tablets 16 grams within the scope of invention (Table 3).

**Table 3. Compositions of dishwasher tablets 16 grams made of the claimed composition.**

| **No** | **Ingredient** | **Formulation No 1** | **Formulation No 2** | **Formulation No 3** | **Formulation No 4** |
|---|---|---|---|---|---|
| | | **Content, % wt.** | | | |
| 1 | Purified water | q.s. | q.s. | q.s. | q.s. |
| 2 | Caustic ash | 23.5 | 23.5 | 23.5 | 23.5 |
| 3 | Sodium percarbonate | 21.0 | 21.0 | 21.0 | 21.0 |
| 4 | Sodium carbonate | 6.5 | 6.5 | 6.5 | 6.5 |
| 5 | Citric acid, anhydrous | 5.0 | 5.0 | 5.0 | 5.0 |
| 6 | Sodium hydrocarbonate | 2.5 | 2.5 | 2.5 | 2.5 |
| 7 | Helating agent MGDA | 5.0 | 5.0 | 5.0 | 5.0 |
| 8 | PEG-4000 | 1.2 | 1.2 | 1.2 | 1.2 |
| 9 | Sodium gluconate | 16.2 | 16.2 | 16.2 | 16.2 |
| 10 | TAED activator | 3.75 | 3.75 | 3.75 | 3.75 |
| 11 | Sodium polyacrylate | 4.35 | 4.35 | 4.35 | 4.35 |
| 12 | Acrylate-copolymer | 0.8 | 0.8 | 0.8 | 0.8 |
| 13 | Polycarboxylates | 4.35 | 4.35 | 4.35 | 4.35 |
| 14 | Low-foaming nonionic alkoxylate-based surfactant | 2.25 | 2.25 | 2.25 | 2.25 |
| 15 | Hydroxypropyl-beta-cyclodextrins | - | - | 0.01 | 0.06 |
| 16 | Manganese salt-based oxidation catalyst (on a pure basis) | - | 0.01 | 0.02 | 0.005 |
| 17 | Biotechnology-derived protease (subtilisin) (on a pure basis) | 0.25 | 0.25 | 0.25 | 0.25 |
| 18 | Biotechnology-derived amylase (on a pure basis) | 0.025 | 0.025 | 0.025 | 0.025 |
| 19 | Pigments for coloring the tablet layers (total) | 0.08 | 0.08 | 0.08 | 0.08 |
| 20 | Perfume agents and essential oils (total) | 0.175 | 0.175 | 0.175 | 0.175 |

### Example 4.

In order to test the efficacy, the studies were performed to evaluate the hygienic cleansing and elimination of foodborne bacteria with the use of the claimed composition in the form of dishwasher tablets (formulations No 1-4). The test microorganisms included standard strains of *Staphylococcus aureus* ATCC^{®}29213, *Escherichia coli* ATCC^{®}25922, *Pseudomonas aeruginosa* ATCC^{®}27853, *Enterococcus faecalis* ATCC^{®}19433, *Klebsiella pneumonia* ATCC^{®}13883, *Salmonella enterica subsp. enterica serovar Typhimurium* ATCC^{®}14028, and also clinical isolates of *Bacillus cereus, Listeria monocytogenes* and *Serratia marcescens,* which are the causative agents of serious gastrointestinal infections and marked indigestion due to poor quality of washing the dishware, kitchen utensils, vegetables and fruit. These bacterial strains are responsible for nutritional disorders and diseases caused by ingestion of nonwashed fruit [Bintsis T., 2017], vegetables [Aurin S. A., et al., 2020] and from the surfaces in contact with the food products, for example, from dishes and cutlery, kitchen boards [Lee J. et al., 2007, Myszka K., 2011, Touimi G. B. et al., 2019], sponges used for washing, and from dishwashers.

LB broth [Sambrook et al., 1989] prepared from ready dry media (20 g/L) with addition of 1.5% agar was used as nutritive medium. The medium was sterilized by autoclaving at 121°C for 20 minutes.

Cell suspensions were diluted in 1 mL of LB medium to optical density of 10⁶ CFU/mL. Dishwashing powders were dissolved in 10 mL of sterile distilled water to final concentration 0.4% (0.4% solution corresponds to dissolution of 20g of dishwasher powder or tablets in 5L of water during the basic cycle of washing). In Eppendorf tubes bacterial suspension (900 µL) was mixed with 100 µL of 0.4% tablet solution.

Selected microorganisms were incubated for 1 hour at temperatures 30°C, 45°C and for 20 minutes at temperature 45°C with or without the tablet. To evaluate antibacterial activity cells were count using serial dilution method. Serial 10-fold dilutions in sterile NaCl solution were prepared from liquid bacterial culture taken from each Eppendorf tube, and 3 µL of cell suspension were transferred on LB agar medium in the dishes. CFU were counted in the drops containing at least 5 colonies multiplying by 7. The experiments were carried out in 5 biological replicates (n=5) and after statistical processing expressed the result as M+SD.

### Results.

Based on the results of evaluation of hygienic effect and elimination of foodborne bacteria, it was established that the test composition at various concentrations in dry tableted formulation for automated dishwashing (formulations No 3 and No 4) produced pronounced hygienic effect against *Salmonella enterica, Escherichia coli, Enterococcus faecalis, Listeria monocytogenes, Bacillus cereus* and *Staphylococcus aureus* under simulated washing cycles at lower temperatures (30°C and 45°C) and short cycles (20 and 60 minutes) compared to the reference formulation free from the composition of the invention (formulation No 1) and compared to the reference formulation which comprised less than all 4 components of the composition (formulation No 2). Specifically, the combination of oxygen bleaching agent, bioprotease stable in the presence of oxidants and metal salts, organic manganese compounds at oxidation state +2 or +3, and hydroxypropyl-beta-cyclodextrins provides considerable reduction of bacterial load and the quickest and the most significant antibacterial effect against the particular strains of foodborne bacteria.

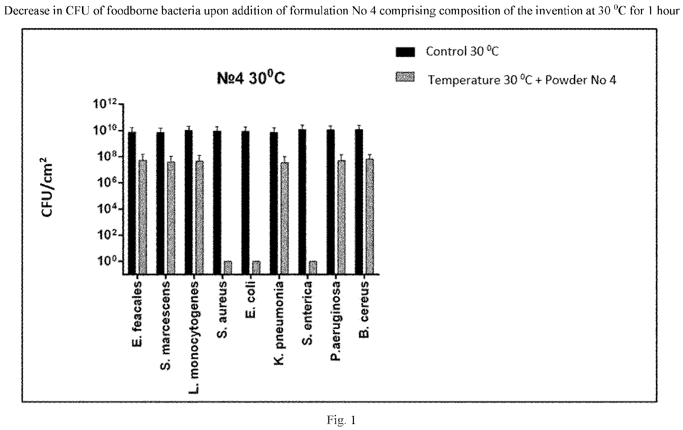

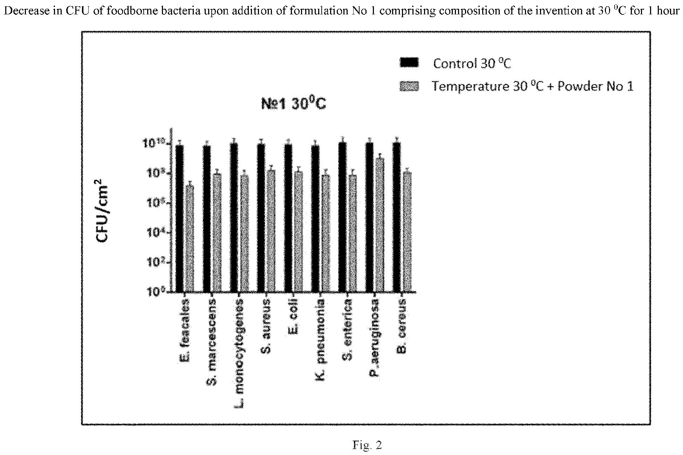

The incubation of bacterial suspension at 30°C for 1 hour in the presence of composition of the invention (formulation No 4) leads to CFU decrease by 2 orders of magnitude against *E*. *faecalis, S. marcescens, L. monocytogenes, K. pneumoniae, P. aeruginosa* and *B. cereus* and by more than 3 orders of magnitude against *S. aureus, E. coli* and *S. enterica,* thus evidencing 99% and 99,9% level of hygienic effect, respectively (Figure 1). Specifically, addition of hydroxypropyl-beta-cyclodextrins provides significant inhibition by 9 orders of magnitude of visible growth of *S. aureus, E. coli* and *S. enterica,* resulting in 99.999% antibacterial effect and protection against food infections even with short cycles at lower temperatures. The use of formulation No 1 free from hydroxypropyl-beta-cyclodextrins and organic manganese complex at oxidation state +2 or +3 does not result in marked hygienic effect against foodborne bacteria - the growth inhibition by 2 or less orders of magnitude is observed; these values correspond to less than 99% effect and do not comply with the requirements of enhanced safety and prevention of gastrointestinal diseases and nutritional disorders (Figure 2).

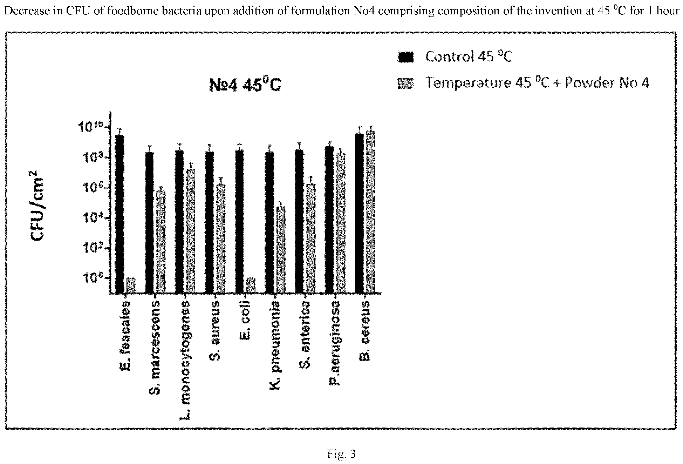

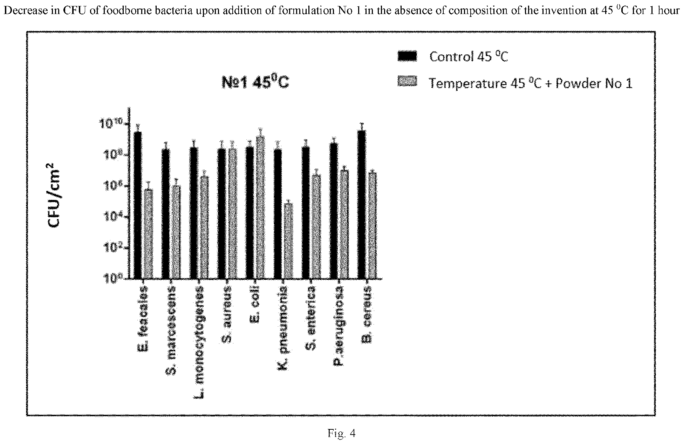

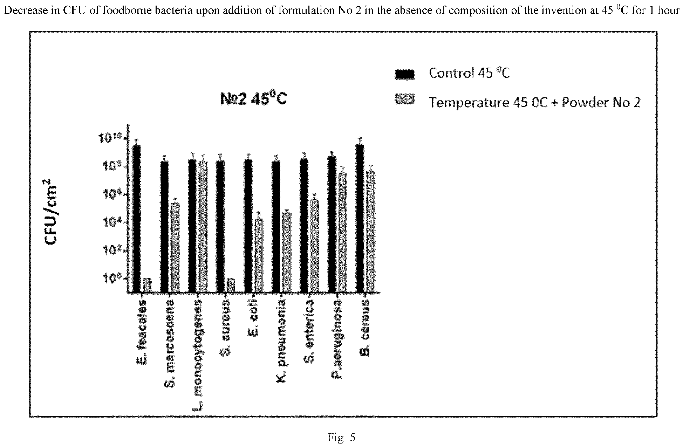

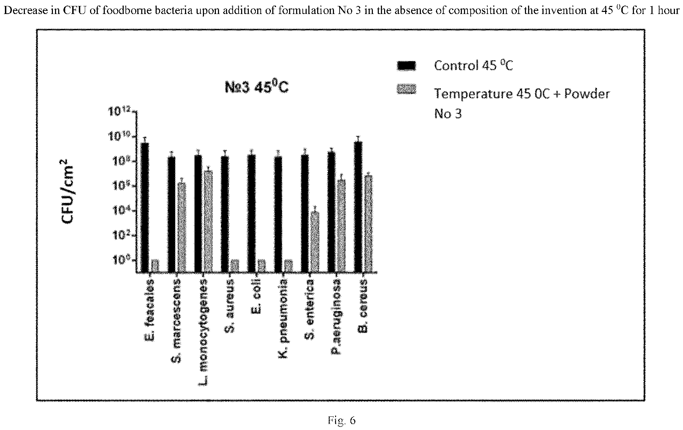

Similar results were obtained with the tablets for automated dishwashing containing the composition of the invention (formulations No 3, 4) after incubation for 1 hour at 45°C. The use of composition of the invention (formulation No 4) for incubation for 1 hour at 45°C, corresponding to short cycle, results in CFU decrease by 2 orders of magnitude against L. *monocytogenes, S. aureus* and *S. enterica* and by more than 3 orders of magnitude against *E*. *faecalis, S. marcescens, E. coli, K. pneumoniae* suggesting 99% and 99.9% hygienic effect respectively (Figure 3). Specifically, addition of hydroxypropyl-beta-cyclodextrins provides significant inhibition by 3-9 orders of magnitude of visible growth of *E*. *faecalis, S. aureus, E. coli, S. marescens* resulting in 99.999% antibacterial effect and protection against food infections even with short cycles at lower temperatures (Figure 3). The use of formulation No 1 free from hydroxypropyl-beta-cyclodextrins and organic manganese complex at oxidation state +2 or +3 does not result in marked hygienic effect against foodborne bacteria except *E*. *faecalis* and *K. Pneumoniae* - the growth inhibition by 2 orders is observed; this value corresponds to less than 99% effect and do not comply with the requirements of enhanced safety and prevention of gastrointestinal diseases and nutritional disorders (Figure 4). Addition of organic manganese complex at oxidation state +2 or +3 to formulation of the invention (formulation No 2) allows to achieve significant inhibition of foodborne bacteria *E. faecalis, S. aureus, E. coli;* however, no significant improvement of the effect against *K. pneumonia, S. enterica, P. aeruginosa* and *B. cereus* is observed (Figure 5). It was unexpectedly established that addition of even small amount of hydroxypropyl-beta-cyclodextrins to composition of the invention significantly improves the hygienic effect against *K. pneumonia, S. enterica, P. aeruginosa* and *B. cereus* (Figure 6) allowing to achieve full coverage and protection against the most common foodborne bacteria by 99.9% and more. Only the presence of oxygen bleaching agent, bioprotease resistant to oxidants and metal salts, organic manganese compounds at oxidation state +2 or +3, and hydroxypropyl-beta-cyclodextrins allows to achieve significant antibacterial effect and risk reduction of indigestion while using the eco-modes, short cycles and cycles at lower temperatures for automated dishwashing.

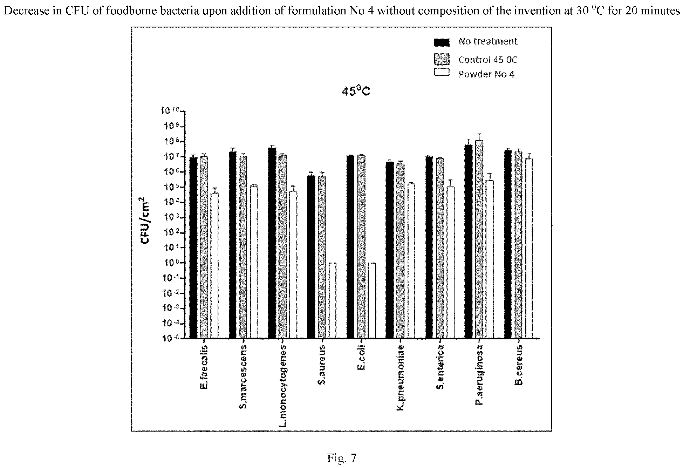

The use of the proposed composition of the invention in dishwasher tablets (formulation No 4) for 20 minutes at 45°C results in significant CFU reduction by 3 orders of magnitude or more (>99.9% of all bacterial cells) for strains: *S. aureus, L. monocytogenes, E. coli, P. aeruginosa* and *S. enterica,* and by 2 orders or more (>99% of all bacterial cells) for strains *E*. *faecalis* and *S. marcescens.* The study results are shown in Figure 7. Instant hygienic cleansing effect allows to reduce microbial burden on human organism and to decrease the risk of food poisoning and nutritional disorders in immunocompromised persons. Only the presence of oxygen bleaching agent, bioprotease resistant to oxidants and metal salts, organic manganese compounds at oxidation state +2 or +3, and hydroxypropyl-beta-cyclodextrins allows to achieve significant antibacterial effect and risk reduction of indigestion while using the extrashort rinsing cycles not intended for intensive washing of dishware and stain removal.

Thus, the composition of invention provided by the Authors offers significant hygienic effect against the most common foodborne bacteria (*Salmonella enterica, Escherichia coli, Enterococcus faecalis, Listeria monocytogenes, Bacillus cereus, Klebsiella pneumonia, Staphylococcus aureus*) both after incubation at 45° for 20 and 60 minutes, and after incubation at 30°C for 1 hour. These experiments demonstrate the best result with respect to health protection against foodborne bacteria of nutritive disorders on dishware, cutlery, kitchen boards and dishwasher. The obtained results are determined by joint contribution of all four components of the composition, providing elimination of >99.9% of all microorganisms, which are the causative agents of numerous foodborne infections, as follows from statistical data and literature.

The described hygienic effect is favorable for human health and helps to get protection from harmful intestinal infections and indigestion even at lower temperature dishwashing modes (30-45°C) and short cycles of dishwater (20 minutes); this makes the proposed composition contributing to lesser burden on the environment rather than just efficient.

Besides the claimed composition, the Authors have also tested the hygienic effects of the other compositions against the selected microorganisms, among them the low dose compositions, cyclodextrin-free compositions, or compositions lacking the active ingredients proposed by the Authors. The experimental results showed that none of the compositions except the claimed one had demonstrated extra pronounced hygienic effect. These results suggest extreme efficacy and synergistic effect of disclosed composition of the invention, said composition based on oxygen bleaching agent, bioprotease stable in the presence of oxidants and metal salts, organic manganese compounds at oxidation state +2 or +3, and hydroxypropyl-beta-cyclodextrins.

### Example 5.

Laboratory test assessed stability and preservation of the composition active oxygen, in presence of various combinations in composition of the invention, in particular, protease (subtilisin), organic manganese compounds and cyclodextrins in dishwasher tablets for automated dishwashing, as shown in Table 4.

The test was performed in laboratory following the internal procedure of the company to assess the active oxygen after storage for 4 weeks in the chamber at temperature 37+3°C and high humidity 60+10%. This test allows to assess the preservation of active oxygen in the finished product using accelerated stability testing under heating, to detect residual activity during the shelf life and, respectively, to assess the product efficacy. Preservation of active oxygen in the presence of atmospheric moisture proves the absence of interaction between sodium percarbonate, manganese compounds as oxidants, and protease, and, respectively, the maintenance of antibacterial effect. The samples of dishwasher tablets for automated dishwashing were prepared, as shown in Table 4, and organic manganese compounds and beta-cyclodextrins were added at several concentrations, either separately or combined. The samples were thermostated in drying oven for 4 weeks, and then the oxygen value was evaluated in each formulation using the developed procedure. This test allows to detect any changes in active oxygen level under any changes. The estimation error for residual oxygen is +0.03%. The tests were carried out in triplicate (n=3).

The sensitive titrimetric method was used to determine an active oxygen level, said method based on oxidation-reduction reaction of potassium permanganate, and hydrogen peroxide release in sulfuric acid environment. The method is also applicable to determination of active oxygen weight fraction in the range from 0.3% to 14.0% in liquids, including viscous liquids, in dishwasher powders, granulates, and tablets. The sample weight is equal to 1 dishwasher tablet. It is allowed to take the sample weight in the amount corresponding to 10 mg of active oxygen. The sample is weighed in a beaker. The result is expressed in grams with the accuracy of four decimal places. The sample weight is quantitatively transferred from the beaker to conical flask with 50-60 m of distilled water. The flask content is mixed manually or on magnetic stirrer. The sample weight is quantitatively transferred from the beaker into volumetric flask with distilled water, diluted to the volume and mixed. Some solution is taken with the pipette, transferred to conical flask, 10-15 mL of sulfuric acid solution is added; the solution is thoroughly mixed and titrated with potassium permanganate solution until pink coloration persists for 1 minute. The second and the third determinations are carried out using new weight of tested formulation. Weight fraction of hydrogen peroxide (X₂, %) is calculated from the equation: X₂ = X₁ x 2,125, wherein X₁ - weight fraction of active oxygen, 2,125 - conversion factor used to convert the active oxygen weight fraction to hydrogen peroxide weight fraction. Sodium percarbonate is an addition compound of hydrogen peroxide and sodium carbonate. Based on molecular formula, pure sodium percarbonate comprises 32.5% of hydrogen peroxide and 67.5% of sodium carbonate (by weight), so sodium percarbonate weight fractions after storage in dry and wet environments can be calculated.

### Results.

Assessment of active oxygen weight fraction showed that the studied composition based on oxygen bleaching agent, oxidation-resistant protease, manganese compounds and modified beta-cyclodextrins allows to retain considerable amount of active oxygen and to preserve it against loss during the storage period in dry and wet conditions (Table 5). The studied composition supplemented with modified beta-cyclodextrins reduces the loss of active oxygen from 33% to 7% when stored under dry conditions and from 63% to 28% under accelerated storage conditions in wet environment. Under wet conditions the combination of sodium percarbonate, protease and manganese compounds show critical loss of active oxygen weight fraction by 63% considerably impairing the degree of removal of colored stain and hygienic effect in the course of automated dishwashing. Addition of modified beta-cyclodextrins even at concentration 0.01% wt. allows to increase the composition stability by 22% under dry conditions and by 37% under wet conditions due to saving manganese compounds and oxygen bleaching agent from premature interaction and auto-oxidation. Increase in manganese compound level does not affect the formulation stability in the presence of modified beta-cyclodextrins. Increase in modified beta-cyclodextrin level enables to reduce the degradation of active oxygen from 15-18% to 7% in dry environment and preserves the formulation stability in wet environment. The test results are demonstrated in Table 5.

**Table 5. Residual composition-derived active oxygen after storage**

| **Test sample** | **Ingredients of the composition** | **Sodium percarbonate weight fraction, %** | | | | |
|---|---|---|---|---|---|---|
| | | **Formulation stability, Week 4** | | | | |
| | | **Baseline control** | **Dry conditions, humidity 20-40%** | | **Wet conditions, humidity 60-70%** | |
| Sample No 1 | Sodium percarbonate 21% | 2.7% | 1.8% | -33% | 1.2% | -55% |
| | Protease (subtilisin) 0.25% | | | | | |
| Sample No2 | Sodium percarbonate 21% | | 1.7% | -37% | 1.0% | -63% |
| | Protease (subtilisin) 0.25% | | | | | |
| | Manganese compound 0.01% | | | | | |
| Sample No3 | Sodium percarbonate 21% | | 2.3% | -15% | 2.0% | -26% |
| | Protease (subtilisin) 0.25% | | | | | |
| | Manganese compound 0.01% | | | | | |
| | Hydroxypropyl-beta-cyclodextrins 0.01% | | | | | |
| Sample No4 | Sodium percarbonate 21% | | 2.2% | -18% | 1.9% | -29% |
| | Protease (subtilisin) 0.25% | | | | | |
| | Manganese compound 0.02% | | | | | |
| | Hydroxypropyl-beta-cyclodextrins 0.01% | | | | | |
| Sample No5 | Sodium percarbonate 21% | | 2.5% | -7% | 2.3% | -28% |
| | Protease (subtilisin) 0.25% | | | | | |
| | Manganese compound 0.005% | | | | | |
| | Hydroxypropyl-beta-cyclodextrins 0.06% | | | | | |
| Sample No6 | Sodium percarbonate 21% | | 2.5% | -7% | 2.3% | -28% |
| | Protease (subtilisin) 0.25% | | | | | |
| | Manganese compound 0.01% | | | | | |
| | Hydroxypropyl-beta-cyclodextrins 0.06% | | | | | |

Composition of dishwasher tablets based on oxygen bleaching agent, oxidant-resistant protease, manganese compounds, and modified beta-cyclodextrins allows to preserve the active oxygen weight fraction, to preserve sodium percarbonate from auto-oxidation by manganese compounds and keep the product stable during the shelf-life in order to achieve high performance and technical result, in particular, pronounced hygienic effect against foodborne bacteria *Salmonella enterica, Escherichia coli, Enterococcus faecalis, Listeria monocytogenes, Klebsiella pneumonia, Bacillus cereus* and *Staphylococcus aureus* at lower washing temperatures.

## Claims

1. A cleaning composition comprising the following components:
A) at least one oxygen bleaching agent;
B) at least one protease;
C) at least one manganese compound; and
D) at least one cyclodextrin.

2. The cleaning composition of claim 1, wherein said bleaching agent is sodium percarbonate.

3. The cleaning composition of claim 1 or claim 2, wherein said protease is subtilisin.

4. The cleaning composition of any of the preceding claims, wherein said manganese compound is an organic complex of manganese salt with an oxidation degree of +2 or +3.

5. The cleaning composition of any of the preceding claims, wherein said cyclodextrin is a beta-cyclodextrin, preferably hydroxypropyl-beta-cyclodextrin.

6. The cleaning composition of any of the preceding claims, wherein the mass ration of said components is the following:
| | | | |
|---|---|---|---|
| A: | B: | C: | D: |
| 15-25 | 0.01-0.5, preferably 0.01-0.25 | 0.001-0.100 | 0.005-0.100 |

7. The cleaning composition of any of the preceding claims, wherein said composition is assembled as a single composition comprising said components A) to D).

8. The cleaning composition of any of the preceding claims, wherein said cleaning composition is a formulation selected from the following: solution, paste, gel, powder, granulate, tablet or capsule encapsulating any of the foregoing formulations.

9. The cleaning composition of any of the preceding claims, wherein said cleaning composition is a cleaning dishwashing composition, preferably an aqueous dishwashing solution.

10. The cleaning composition of claim 9, wherein said cleaning dishwashing composition or aqueous dishwashing solution is for manual hand dishwashing.

11. The cleaning composition of claim 9, wherein said cleaning dishwashing composition or aqueous dishwashing solution is for automatic machine dishwashing, preferably for automatic machine dishwashing, further preferably for automatic machine dishwashing as per an eco-wash program in an automatic machine dishwasher.

12. Use of the cleaning composition of any of claims 1-11 for
i) antibacterial treatment of non-living objects and their surface, wherein said bacteria are preferably selected from the group consisting of: *Salmonella enterica, Escherichia coli, Enterococcus faecalis, Listeria monocytogenes, Bacillus cereus* and *Staphylococcus aureus,*
and/or
ii) manual hand and/or automatic machine dishwashing, preferably for automatic machine dishwashing, further preferably for automatic machine dishwashing as per an eco-wash program in an automatic machine dishwasher.

13. The use of claim 12, wherein said cleaning composition is applied to the non-living object's surface and/or dishwashing items as per an aqueous solution in manual hand washing at following parameters:
- at water hardness less than 42° dH, preferably 0-28° dH,
- at any temperature from +10°C to +60°C, preferably +20°C to +40°C or +10°C to +30°C,
- at pH=7-12, preferably at pH=10-12.

14. The use of claim 12, wherein said cleaning composition is applied to the non-living object's surface and/or dishwashing items as per an aqueous solution in automatic machine washing at the following parameters:
- at water hardness less than 42 dH, preferably 0-28° dH,
- at any washing temperature from +20°C to +60°C, 30°C to +50°C, +20°C to +40°C, +40°C to +45°C, or +35°C to +45°C, and
- at pH=7.0-12, preferably 8.0-12, further preferably 9.0-11.5, further preferably 9.5-11.5 or 10-12, preferably 10-11. further preferably 10-10.5.

15. The use of subtilisin for the stabilization of a manganese compound in a composition comprising said manganese compound and further comprising sodium percarbonate and a protease, preferably for the stabilization of a composition as defined in any of claims 1-11 and further preferably for the stabilization of said composition providing for the use of said composition according to claims 12-14.
